(19) Europäisches Patentamt
European Patent Office
Office européen des brevets

(11) Publication number: **0 116 422**
**B1**

(12) # EUROPEAN PATENT SPECIFICATION

(45) Date of publication of patent specification: **06.04.88**

(51) Int. Cl.$^4$: **A 61 K 7/06,** A 61 K 7/08, C 11 D 3/06, C 11 D 17/00

(21) Application number: **84300440.9**

(22) Date of filing: **25.01.84**

(54) **Liquid cleansing compositions.**

(30) Priority: **03.02.83 GB 8303014**

(43) Date of publication of application:
**22.08.84 Bulletin 84/34**

(45) Publication of the grant of the patent:
**06.04.88 Bulletin 88/14**

(84) Designated Contracting States:
**AT BE CH DE FR IT LI LU NL SE**

(56) References cited:
**DE-A-1 617 327**
**FR-A- 857 207**
**US-A-3 810 478**
**US-A-4 107 067**
**US-A-4 348 292**

**The file contains technical information submitted after the application was filed and not included in this specification**

(73) Proprietor: **RECKITT AND COLMAN PRODUCTS LIMITED**
**P.O. Box 26 1-17, Burlington Lane**
**London W4 2RW (GB)**

(72) Inventor: **Harmer, Jane**
**2 Jessop Avenue**
**Almondbury Huddersfield (GB)**
Inventor: **Cook, Robert Victor**
**11 Greenleas**
**Aston on Trent Derbyshire (GB)**

(74) Representative: **Allard, Susan Joyce et al**
**BOULT, WADE & TENNANT 27 Furnival Street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 116 422 B1

Courier Press, Leamington Spa, England.

**Description**

This invention relates to multilayer liquid cleansing compositions in which one liquid phase is dispersible in another liquid phase upon shaking the composition and which on standing separate out into distinct layers. More particularly the invention relates to shampoo compositions for use on the body and hair.

Multilayer cleansing compositions have been proposed in which one layer is miscible in all proportions with water whilst another layer being of an oily nature is immiscible with water. The oily layer may comprise paraffin oil, certain esters or silicone oils. Typically the lower layer comprises a water-miscible layer including water as solvent and containing dissolved detergent whilst a water-immiscible oil-rich phase floats on the aqueous phase. Examples of such compositions include bath shampoos which are added to bath water to afford body cleansing and which provide a film of oil which is retained on the skin upon emerging from the bath to condition and/or perfume the skin; and hair shampoos where the oily layer serves to condition and/or groom the hair. An Example of such a hair shampoo occurs in United States of America Specification US—A—3810478 in which the upper layer relies for its integrity upon a major amount of a mineral oil. A disadvantage of such oily materials in formulations is that they can leave an unsightly ring on the bath after use and in hair shampoos for use on naturally greasy heads are not necessarily a desirable component.

United States Patent US—A—4348292 described multilayer compositions for heavy duty cleaning in the form of a single-dose concentrate for addition to water to provide a cleansing solution or dispersion. The concentrate has two aqueous layers, one layer containing at least one surfactant and the other layer containing at least one detergent builder. Preferred compositions were those in which the surfactant layer contains 50—90% by weight of surfactant and the detergent builder layer contains 30—80% by weight of detergent builder. These compositions are claimed to have enhanced cleansing efficacy as compared with homogeneous compositions, however, there is no guidance given on other than single dose presentations. In stepping from a single-dose unit to a multidose unit problems can arise in ensuring a uniform fill of each phase into the container and more importantly, in the case of a shampoo composition in ensuring that the proper dose of each phase is dispensed from the container when in use.

The specification of West German Patent Application DE—A—1617327 discloses a hair treatment composition containing 0.05 to 2.0% of certain metal polyphosphates of formula $M_{n+2}P_nO_{3n+1}$, wherein n is unspecified and M represents an alkali metal ion. Such compositions are intended to be applied to hair newly washed with soapless detergent, rinsed with water and squeeze-dried and impart conditioning effects. The specification is emphatic that the treatment is conducted as a separate step and no further rinsing is to be carried out before drying or setting. Furthermore, the specification advises that only ineffective amounts of the generically disclosed polyphosphates may be included into a hair shampoo formulation. A disadvantage of this disclosure is the necessity for a separate "conditioner" rinse step.

During our investigations into shampoo compositions suitable for use by those persons desirous of frequent washing of their hair, compositions were developed which were two phase and which contained a variety of detergent builders. Most unexpectedly it was found that compositions containing sodium hexametaphosphate as the detergent builder exhibit conditioning effects on hair. Moreover such compositions may be used in a conventional manner, that is, simply applied to the wetted hair then worked up to a lather and rinsed out with a copious supply of clear water. Thus the feel and the manageability of hair so washed is improved.

Accordingly the present invention provides a liquid shampoo composition having an upper aqueous layer and a lower aqueous layer, which are temporarily dispersible one in the other upon agitation and wherein each layer is miscible with water in substantially all proportions, said upper layer having dissolved therein 8% to 25% by weight of the composition of at least one detergent and said lower layer containing at least 6% by weight of the composition of sodium hexametaphosphate dissolved therein.

Preferably, the dispersion resulting from the agitation of the layers is stable for sufficient time to allow uniform filling of the compositions into containers so that on standing the relative ratios of the phases within the containers are constant. This avoids the problem of having to fill each container separately with predetermined amounts of two different concentrates. Preferably, the dispersion resulting from the agitation of the two layers has a pH of from 6 to 7.

Desirably each of the upper and lower layers is substantially transparent. As will be discussed in detail later the relative ratios of the phases is dependent upon the nature and amounts of various components present.

Sodium hexametaphosphate is the term applied to a mixture of condensed phosphates of the formula:—

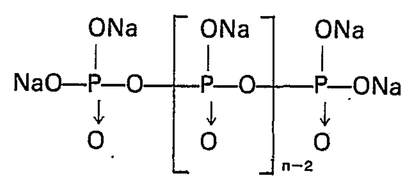

in which the average value of n is about 12, that is a selection from the generic disclosure of West German Specification DE—A—1617327 of the case that n=12.

In the compositions the amount of sodium hexametaphosphate will normally be at least 6% by weight of the composition.

Amounts of other detergent builder salts such as ammonium, sodium or potassium chlorides, sulphates, metaphosphates and/or ortho-phosphates may be included in the compositions. The total weight of the sodium hexametaphosphate plus other detergent builder salt(s) (if present) will normally comprise from 8 to 25% by weight of the composition and preferably 14—20%.

The detergent may be an anionic, cationic, amphoteric or nonionic detergent or mixtures thereof. Preferably the compositions will include at least one anionic detergent.

The concentration of detergent in the compositions of the present invention is generally similar to those in conventional shampoo compositions.

Typical of the detergents that may be used are: ammonium, substituted ammonium or sodium lauryl sulphates, or ethoxylated derivatives thereof containing up to 6 ethoxyl groups per mole; substituted ammonium such as the tri- and/or diethanolamine salts of sulphated ethoxylated coconut monoethanolamide containing up to 6 ethoxyl groups per mole; α-olefin sulphates and alkyl aryl sulphonates of which the high-foaming members are preferred; imidazoline betaine or cetyltrimethyl ammonium bromide. The concentration of these components is conveniently in the range 8 to 25% by weight based on the total composition, and preferably in the range 10% to 18%.

In addition to the essential components mentioned above the compositions will normally include a foam boosting agent such as coconut diethanolamide and optionally a superfatting agent such as ethoxylated coconut monoethanolamide. The quantities of these two components can have an effect on the relative phase volumes and the viscosity of the compositions.

The choice of detergent(s) in part controls the phase volume ratio of the final product. Thus, the chemical nature and the relative concentrations of the detergents employed influence the phase volume ratio. The phase volumes are also dependent upon the amount of the sodium hexametaphosphate and the amount and/or nature of any additional detergent builder salt. For example when the detergent is an alkyl sulphate salt and is employed in a fixed concentration of active principle in a given detergent system, a certain phase volume ratio is attained. If the anion moiety of the surfactant is ethoxylated thereby to increase the solubility thereof with the same cation, the phase volume ratio is increased that is to say that relative volume of the lower layer increases.

In the case, however, if for a particular unethoxylated anionic surfactant the nature of the cation is altered so to increase the water-solubility of the surfactant then the phase volume ratio decreases that is to say the relative volume of the lower layer decreases and in the limit it tends to disappear resulting in a homogeneous mixture. This has been found for example in the series:— sodium alkyl sulphate; ammonium alkyl sulphate; diethanolamine alkyl sulphate; triethanolamine alkyl sulphate in which series water-solubility increases from start to finish. This is a basis for finely adjusting the phase volume ratio to a predetermined value.

In order to effect an improved aesthetic appearance to the compositions one or more colouring materials may be included so that the different layers assume different colours or shades of colour to give the compositions a pleasing appearance.

The compositions may also include medicaments such as coal tar or additional conditioning agents such as solubilised lanolin or hydrolysed proteins. The compositions may include special treatment materials which may be solids, semisolids or liquids to achieve special effects. For example an oily material or ester such as isopropyl myristate may be included as a separate phase to form a multilayer product, or combined with one of the upper or lower layer phases if it is desired to produce a two layer product.

A further feature of shampoo compositions in general is that the public has grown accustomed to a degree of "thickness" consequently compositions need to have controllable viscosity of an order that may be varied to taste. A preferred range is represented by the viscosity range 1—3 Pas (1000—3000 centipoises) as measured with Brookfield viscometer RVT using No. 3 spindle at speed 20 at 20°C. Clearly there may be considerable variations on both sides of this range to suit individual product requirements.

The compositions may include a proportion of an alcohol such as isopropanol or ethanol in relatively low concentrations, of the order 2% w/w of the total composition or less, for example to solubilise a treatment compound such as coal tar, or to solubilise a perfume.

To safeguard against decomposition of components in the presence of heavy metal ions, a strong sequestering agent therefor may be incorporated without seriously disturbing the phase volume ratio. Suitable sequestering aids include ethylenediaminetetraacetic acid; the aminomethylene phosphonates such as those manufactured by Albright and Wilson under their trade mark Briquest, for example acetoethylenediaminetetramethylenephosphonic acid, acetodiphosphonic acid and trinitriloacetic acid. The free acid may be added as such or it may be added in the form of an alkali metal salt. The amount of sequestering aid employed is of the order up to 1.00% w/w of the total composition expressed as free acid, preferably about 0.5% w/w.

The invention is illustrated by the following Examples in which percentages are weight/weight.

Example 1

A hair shampoo was prepared having the following composition:

| | % |
|---|---|
| Triethanolamine lauryl sulphate (40% aqueous solution) | 26.88 |
| Ethoxylated cocomonoethanolamide sulphate triethanolamine salt (40% aqueous solution) | 8.75 |
| Coconut diethanolamide | 2.50 |
| Ethoxylated coconut monoethanolamide | 1.00 |
| Colour, perfume | qs |
| Sodium hexametaphosphate (40% aqueous solution) | 35.00 |
| Water | balance to 100% |

The composition was prepared by mixing thoroughly together with stirring all the components with the exception of coconut diethanolamide, ethoxylated cocomonoethanolamide and perfume which were preblended prior to addition to the rest of the components. The composition was filled directly into bottles whilst maintaining the stirring or the final filling operation can be from a well stirred reservoir. The ratio of the volumes of the upper phase to the lower was 6:4. The viscosity of the shampoo was 1.75 Pas (1750 centipoises).

Examples 2—7

Shampoos of the following compositions were prepared by the method of Example 1.

| | 2 % | 3 % | 4 % | 5 % | 6 % | 7 % |
|---|---|---|---|---|---|---|
| Triethanolamine lauryl sulphate (40% aqueous solution) | 23.57 | — | — | — | 26.0 | — |
| Ethoxylated cocomonoethanolamide sulphate—triethanolamine salt (40% aqueous solution) | 8.9 | — | — | — | 8.0 | — |
| Coconut diethanolamide | 3.0 | 2.5 | 3.0 | 3.0 | 2.5 | 3.5 |
| Sodium hexametaphosphate (40% aqueous solution) | 18.0 | 40.0 | 40.0 | 40.0 | 20.0 | 35.0 |
| Trisodium phosphate | 0.1 | — | — | — | 8.0 | — |
| Sodium dihydrogenphosphate | 10.0 | — | — | — | — | — |
| Colour, perfume | qs | qs | qs | qs | qs | qs |
| Sodium hydroxide | qs to pH 7.0 | — | — | — | — | — |
| Cetyltrimethylammonium bromide | — | 14.0 | — | — | — | — |
| Ethoxylated coconut monoethanolamide | — | 1.0 | 0.75 | 0.75 | 1.0 | 2.0 |
| Diethanolamine lauryl sulphate (33% aqueous solution) | — | — | 42.65 | — | — | — |
| Dodecylbenzenesulphonate—triethanolamine salt (60% aqueous solution) | — | — | — | 24.17 | — | — |
| Imidazoline betaine (40% aqueous solution) | — | — | — | — | — | 36.0 |
| Water | ——balance to 100—— | | | | | |

The seven Examples afford mild shampoos with gentle cleansing agents which cleanse the hair without stripping it of its natural oil and which contain non oily conditioners to maintain its condition. They are also useful for people who wash their hair very frequently and only a single application is necessary. Thus excessive defatting of the hair and/or skin is obviated.

Examples 8, 9

Shampoos of the following compositions were prepared by the method of Example 1.

| | 8<br>% w/w | 9<br>% w/w |
|---|---|---|
| Triethanolamine lauryl sulphate | 26.9 | 26.9 |
| Acylaminopolyglycolether sulphate, triethanolamine salt | 8.8 | 8.8 |
| Coconut diethanolamide | 2.5 | 2.5 |
| Ethoxylated coconut monoethanolamide | 1.0 | 1.0 |
| Sodium hexametaphosphate | 16.0 | 16.0 |
| Acetodiphosphonic acid | 0.5 | — |
| Ethylenediaminetetracetic acid | — | 0.5 |
| Perfume | qs | qs |
| Colourant | qs | qs |
| Preservative | qs | qs |
| Water | ad 100% | ad 100% |

These Examples produce mild shampoo compositions of pH about 6.5, the components of which are at acceptable concentrations and the compositions are of a viscosity to be expected by users. They also are useful for people who wash their hair very frequently and only a single application is necessary at each washing. Thus excessive defatting of the hair and/or skin is obviated.

**Claims**

1. A liquid shampoo composition having an upper aqueous layer and a lower aqueous layer, which are temporarily dispersible one in the other upon agitation and wherein each layer is miscible with water in substantially all proportions, said upper layer having dissolved therein 8% to 25% by weight of the composition of at least one detergent and said lower layer containing at least 6% by weight of the composition of sodium hexametaphosphate dissolved therein.

2. A shampoo composition according to claim 1 wherein a detergent builder salt selected from ammonium, sodium or potassium chlorides, sulphates, metaphosphates or orthophosphates is present in amount such that together with the sodium hexametaphosphate it represents 8% to 25% by weight of the composition.

3. A shampoo composition as claimed in claim 2 wherein the total weight of sodium hexametaphosphate and optional detergent builder salt(s) is from 14 to 20% by weight of the composition.

4. A shampoo composition as claimed in any preceding claim wherein the detergent is selected from ammonium, substituted ammonium or sodium lauryl sulphates, or ethoxylated derivatives thereof containing up to 6 ethoxyl groups per mole; substituted ammonium such as the tri- and/or diethanolamide salts of sulphated ethoxylated coconut monoethanolamide containing up to 6 ethoxyl groups per mole; α-olefin sulphates and alkyl aryl sulphonates; imidazoline betaine or cetyltrimethyl ammonium bromide.

5. A shampoo composition as claimed in any one of the preceding claims wherein the total weight of the detergent(s) is from 10 to 18% by weight of the composition.

6. A shampoo composition as claimed in any one of the preceding claims wherein the dispersion resulting from the agitation of the layers has a viscosity of up to 3 Pas (3000 centipoises) when measured on a Brookfield viscometer model RVT using spindle No. 3 at speed 20 at 20°C.

7. A shampoo composition as claimed in claim 6 wherein the viscosity of the said dispersion is in the range of from 1 to 3 Pas (1000 to 3000 centipoises).

8. A shampoo composition as claimed in any one of the preceding claims wherein the dispersion resulting from the agitation of the layers has a pH of from 6 to 7.

9. A shampoo composition as claimed in any one of the preceding claims which additionally contains a sequestering agent which is distributed between the two layers.

10. A shampoo composition as claimed in claim 9 wherein the sequestering agent is ethylenediamine tetracetic acid or an aminomethylene phosphonate.

11. A method of washing hair which comprises shaking a shampoo composition as claimed in any one of the preceding claims to form a temporary dispersion, applying said so shaken composition to moistened hair, working to a lather and rinsing with clear water whereby the hair is both washed and conditioned.

12. A method for the preparation of a shampoo composition as claimed in any one of Claims 1 to 10 which comprises blending together at least one detergent in an amount to provide from 8% to 25% by weight of the total composition, sodium hexametaphosphate in an amount to provide at least 6% by weight of the total composition and any other desired ingredients, adding water in the amount required to bring the composition to 100% by weight, stirring the mixture of components until a uniform dispersion is obtained and filling the so-produced composition into containers.

13. A method for the preparation of a shampoo composition as claimed in any one of Claims 1 to 10 which comprises blending a 40% aqueous solution of at least one detergent and a 40% aqueous solution of sodium hexametaphosphate, optionally with the addition of any other desired ingredients, with water to form a uniform dispersion, the detergent forming 8% to 25% by weight of the total composition and the sodium hexametaphosphate forming at least 6% by weight of the total composition, and filling the so-produced composition into containers.

**Patentansprüche**

1. Flüssige Shampoo-Zusammensetzung mit einer oberen wässrigen Schicht und einer unteren wässrigen Schicht, die zeitweise durch Schütteln ineinander dispergierbar sind und wobei jede Schicht mit Wasser in im wesentlichen jedem Verhältnis mischbar ist, wobei die obere Schicht gelöst 8 bis 25 Gew.-% der Zusammensetzung mindestens eines Detergens enthält und die untere Schicht mindestens 6 Gew.-% der Zusammensetzung an Natriumhexametaphosphat gelöst enthält.

2. Shampoo-Zusammensetzung nach Anspruch 1, worin ein Detergens-bildendes Salz, ausgewählt aus Ammonium-, Natrium- oder Kaliumchloriden, -sulfaten, -metaphosphaten oder -orthophosphaten, in einer solchen Menge vorhanden ist, daß es zusammen mit dem Natriumhexametaphosphat 8 bis 25 Gew.-% der Zusammensetzung darstellt.

3. Shampoo-Zusammensetzung nach Anspruch 2, worin das Gesamtgewicht an Natriumhexametaphosphat und gegebenenfalls Detergens-bildendem Salz(en) zwischen 14 und 20 Gew.-% der Zusammensetzung liegt.

4. Shampoo-Zusammensetzung nach einem der vorhergehenden Ansprüche, worin das Detergens ausgewählt ist aus Ammonium-, substituierten Ammonium- oder Natriumlaurylsulfaten, oder ethoxylierten Derivaten davon, enthaltend bis zu 6 Ethoxylgruppen pro Mol; substituiertem Ammonium, wie Tri- und/oder Diethanolamidsalzen von sulfatiertem, ethoxyliertem Kokosnuß-Monoethanolamid, enthaltend bis zu 6 Ethoxylgruppen pro Mol; α-Olefinsulfaten und Alkylarylsulfonaten; Imidazolinbetain oder Cetyltrimethylammoniumbromid.

5. Shampoo-Zusammensetzung nach einem der vorhergehenden Ansprüche, worin das Gesamtgewicht des Detergens (der Detergentien) 10 bis 18 Gew.-% der Zusammensetzung beträgt.

6. Shampoo-Zusammensetzung nach einem der vorhergehenden Ansprüche, worin die Dispersion, die durch Schütteln der Schichten entsteht, eine Viskosität von bis zu 3 Pas (3000 Centipoises), gemessen an einem Brookfield Viskometer Modell RVT unter Benutzung einer Spindel Nr. 3 bei Geschwindigkeit 20 bei 20°C, aufweist.

7. Shampoo-Zusammensetzung nach Anspruch 6, worin die Viskosität der Dispersion im Bereich von 1 bis 3 Pas (1000 bis 3000 Centipoises) liegt.

8. Shampoo-Zusammensetzung nach einem der vorhergehenden Ansprüche, worin die Dispersion, die durch das Schütteln der Schichten entsteht, einen pH von 6 bis 7 aufweist.

9. Shampoo-Zusammensetzung nach einem der vorhergehenden Ansprüche, die zusätzlich ein Trennmittel enthält, das zwischen den beiden Schichten verteilt ist.

10. Shampoo-Zusammensetzung nach Anspruch 9, worin das Trennmittel Ethylendiamintetraessigsäure oder ein Aminomethylenphosphonat ist.

11. Verfahren zum Waschen von Haaren, umfassend Schütteln einer Shampoo-Zusammensetzung nach einem der vorhergehenden Ansprüche unter Bildung einer temporären Dispersion, Auftragen der geschüttelten Zusammensetzung auf angefeuchtetes Haar, Bearbeiten bis Schaum entsteht und Spülen mit klarem Wasser, wodurch das Haar sowohl gewaschen als auch konditioniert wird.

12. Verfahren zur Herstellung einer Shampoo-Zusammensetzung nach einem der Ansprüche 1 bis 10, umfassned Vermischen mindestens eines Detergens in einer Menge, die mindestens 8 bis 25 Gew.-% der Gesamtzusammensetzung ausmacht, mit Natriumhexametaphosphat in einer Menge, die mindestens 6 Gew.-% der Gesamtzusammensetzung ausmacht und jeglichen anderen gewünschten Inhaltsstoffen, Zugeben von Wasser in der Menge, die benötigt wird, um die Zusammensetzung auf 100 Gew.-% zu

bringen, Rühren der Mischung der Komponenten bis eine gleichmäßige Dispersion erhalten wird und Abfüllen der so hergestellten Zusammensetzung in Behälter.

13. Verfahren zur Herstellung einer Shampoo-Zusammensetzung nach einem der Ansprüche 1 bis 10, umfassend Mischen einer 40 %-igen wässrigen Lösung mindestens eines Detergens und eine 40 %-ige wässrige Lösung von Natriumhexametaphosphat, gegebenenfalls unter Zugabe jeglicher anderer gewünschter Inhaltsstoffe, mit Wasser, um eine gleichmäßige Dispersion zu bilden, wobei das Detergens 8 bis 25 Gew.-% der Gesamtzusammensetzung und das Natriumhexametaphosphat mindestens 6 Gew.-% der Gesamtzusammensetzung darstellt, und Abfüllen der so hergestellten Zusammensetzung in Behälter.

**Revendications**

1. Composition de shampooing liquide ayant une couche aqueuse supérieure et une couche aqueuse inférieure qui peuvent être temporairement dispersées l'une dans l'autre par agitation, et dans laquelle chaque couche est miscible à l'eau sensiblement en toutes proportions, ladite couche supérieure ayant dissous au moins un détergent dans un rapport de 8% à 25% en poids de la composition et ladite couche inférieure contenant de l'hexamétaphosphate de sodium dissous dans un rapport d'au moins 6% en poids de la composition.

2. Composition de shampooing selon la revendication 1, dans laquelle un sel adjuvant de détergent choisi parmi les chlorures, les sulfates, les métaphosphates ou les orthophosphates d'ammonium, de sodium, ou de potassium est présent en une quantité telle, qu'ensemble avec l'hexamétaphosphate de sodium ils représentent 8% à 25% en poids de la composition.

3. Composition de shampooing, comme revendiquée dans la revendication 2, dans laquelle le poids total d'hexamétaphosphate de sodium et du ou des sels éventuels adjuvants de détergent est de 14 à 20% en poids de la composition.

4. Composition de shampooing, comme revendiquée dans l'une quelconque des revendications précédentes, dans laquelle le détergent est choisi parmi les sulfates de lauryl, d'ammonium, d'ammonium substitués ou de sodium, ou les dérivés éthoxylés de ceux-ci, contenant jusqu'à six groupes éthoxyle par mole; les sels d'ammonium substitués, tels que les sels de tri- et/ou de diéthanolamide de monoéthanolamide de coco sulfaté et éthoxylés contenant jusqu'à 6 groupes éthoxyle par mole; les sulfates α-oléfiniques et les sulphonates aryl alkyl; la bétaîne imidazoline ou le bromure de cétyltriméthyl ammonium.

5. Composition de shampooing comme revendiquée dans l'une quelconque des revendications précédentes, dans laquelle le poids total du ou des détergents est de 10 à 18% en poids de la composition.

6. Composition de shampooing, comme revendiquée dans l'une quelconque des revendications précédentes, dans laquelle la dispersion résultante de l'agitation des couches a une viscosité allant jusqu'à 3 Pa . s (3 000 centipoises) par mesure à l'aide d'un viscosimètre Brookfield modèle RVT utilisant le pivot No. 3 à la vitesse 20 à 20°C.

7. Composition de shampooing, comme revendiquée dans la revendication 6 dans laquelle la viscosité de ladite dispersion est dans l'intervalle de 1 à 3 Pa . s (1 000 à 3 000 centipoises).

8. Composition de shampooing, comme revendiqué dans l'une quelconque des revendications précédentes, dans laquelle la dispersion résultant de l'agitation des couches a un pH de 6 à 7.

9. Composition de shampooing, comme revendiquée dans l'une quelconque des revendications précédentes, qui contient en outre un agent complexant qui est distribué entre les deux couches.

10. Composition de shampooing comme revendiqué dans la revendication 9, dans laquelle l'agent complexant est l'acide éthylènediamine tétracétique ou un phosphonate aminométhylène.

11. Procédé de lavage de cheveux qui comprend le secouage d'une composition de shampooing, comme revendiquée dans l'une quelconque des revendications précédentes, pour former une dispersion temporaire, l'application de ladite composition secouée à des cheveux humidifiés, une mise en mousse et un rinçage à l'eau claire de telle manière que les cheveux sont à la fois lavés et lotionnés.

12. Procédé de préparation d'une composition de shampooing, comme revendiquée dans l'une quelconque des revendications 1 à 10, qui comprend le mélange d'au moins un détergent en une quantité allant de 8% à 25% en poids de la composition totale avec de l'hexamétaphosphate de sodium en une quantité allant d'au moins 6% en poids de la composition totale et avec tous autres ingrédients souhaités, l'ajout d'eau en une quantité requise pour amener la composition à 100% en poids, l'agitation du mélange de composés jusqu'à l'obtention d'une dispersion homogène et le remplissage de containers avec ladite composition ainsi produite.

13. Procédé de préparation d'une composition de shampooing comme revendiqué dans l'une quelconque des revendications 1 à 10, qui comprend le mélange d'une solution aqueuse à 40% d'au moins un détergent avec une solution aqueuse à 40% d'hexamétaphosphate de sodium, éventuellement avec l'addition de tous autres ingrédients souhaités, avec de l'eau pour former une dispersion homogène, le détergent formant 8% à 25% en poids de la composition totale, et l'hexamétaphosphate de sodium formant au moins 6% en poids de la composition totale et le remplissage de containers avec la composition ainsi produite.